# EUROPEAN PATENT SPECIFICATION

(11) **EP 2 155 038 B1**
(45) Date of publication and mention of the grant of the patent: **26.10.2011**
(21) Application number: 08780612.1
(22) Date of filing: 09.05.2008
(51) Int. Cl.: A61B 1/01

(54) **MEDICAL RETRACTOR AND STABILIZING ASSEMBLY**
MEDIZINISCHER RETRAKTOR UND STABILISIERUNGSANORDNUNG
RÉTRACTEUR MÉDICAL ET ENSEMBLE DE STABILISATION

(30) Priority: 17.05.2007 US 924504 P; 08.04.2008 US 78956
(43) Date of publication of application: 24.02.2010
(73) Proprietor: Boston Scientific Scimed, Inc., Maple Grove, MN 55311-1566 (US)
(72) Inventor: SMITH, Paul, J., Smithfield, RI 02917 (US); WEITZNER, Barry, Acton, MA 01729 (US); KRUEGER, Katie, Merrimack, NH 03054 (US); GOLDEN, John, B., Norton, MA 02766 (US)
(74) Representative: Hauck Patent- und Rechtsanwälte
(86) International application number: PCT/US2008/063192
(87) International publication number: WO 2008/144254

(56) References cited:
- EP-A- 1 749 479
- US-A- 4 608 965
- US-A- 5 275 610
- US-A- 5 730 725
- US-A- 5 803 902
- US-A1- 2004 210 116

## Description

### DESCRIPTION OF THE INVENTION

### Field of the Invention

Embodiments of the present invention relate to endoscopic guide tubes. In particular, exemplary embodiments of the present invention relate to endoscopic guide tubes that are movable with respect to a stabilizing assembly and that are configured to retract tissue. The present disclosure also covers methods of using such devices.

### Background of the Invention

In general, it is desirable to minimize the invasiveness of medical procedures. Invasive medical procedures are generally more expensive, and there is generally a greater risk of complication and discomfort for the patient. For example, open surgery is an invasive medical procedure with significant attendant risks. Since the performance of open surgery typically requires relatively large incisions, relatively large amounts of blood may be lost, the risk of infection may increase, and the potential for post-operative hernias may be higher. Furthermore, relatively large incisions require extended recovery times to allow the incisions to heal.

Laparoscopic procedures are generally less invasive than open surgery. Percutaneous endoscopic gastronomy (PEG) is a laparoscopic procedure that involves incisions through the skin to access various bodily organs. For example, PEG may provide access through the skin to the stomach to allow insertion of a feeding tube for feeding patients who cannot ingest food on their own. The incisions, however, may lead to a risk of infection and other negative effects, such as sepsis, which can be caused by leakage of septic fluid contained in the stomach.

Another type of surgical procedure is a transluminal endoscopic surgical procedure, which may allow access to various bodily organs without having to puncture the skin, which may lead to infection due to exposure to the outside environment. In transluminal endoscopic surgical procedures, an endoscope may be introduced into the body, for example, through a body orifice (e.g., the rectum or mouth). The endoscope is a flexible instrument introduced into the body to access the inside of the body. A light source and a camera may be provided at a distal end (i.e., the end which is inside the body) of the endoscope.

An endoscopic instrument may be used to puncture the wall of the stomach or other organ to allow the endoscope to advance into the abdominal cavity where remotely controlled surgical tools may be used to perform surgical procedures. However, the form of the endoscope and the associated instruments may impose physical limits on the surgical task that can be accomplished. These limitations, in some cases, may restrict endoscopic procedures from producing the same anatomical outcome as conventional surgery. For example, there is limited space within the abdominal cavity between the stomach and the wall of the abdominal cavity. Therefore, the endoscopic instruments must be smaller, and there is limited maneuverability of the working ends of the endoscope and endoscopic instruments.

U.S. Patent No. 5,275,610 describes a surgical retractor for separating body parts during a surgical procedure to thereby create or maintain a surgical cavity. The retractor, however, may not be securable in position with respect to the patient and therefore may inadvertently move out of a desired position during the surgical procedure.

### SUMMARY OF THE INVENTION

The present disclosure is directed to a medical device including an elongated member and a retractor assembly disposed on the elongated member near a distal end of the elongated member. The retractor assembly is configured to change from a collapsed configuration to an expanded configuration, and the retractor assembly is configured to be movable within a body cavity when the retractor assembly is in an expanded configuration.

The medical device comprises furthermore a stabilizing assembly configured to be releasably secured to the elongated member near a proximal end of the elongated member, the stabilizing assembly including a portion capable of being secured to a patient.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate several embodiments of the invention and together with the description, serve to explain the principles of the invention.

Fig. 1 is a schematic view of a guide tube in a patient, the guide tube including a retractor assembly in a collapsed configuration, according to an exemplary embodiment of the invention;

Fig. 2 is a schematic view of the exemplary guide tube of Fig. 1 with the retractor assembly in an expanded configuration;

Fig. 3 is a schematic view of the exemplary guide tube of Fig. 2 with the expanded retractor assembly in a retracted position;

Fig. 4 is a schematic view of the exemplary retractor assembly of Fig. 1;

Fig. 5 is a schematic view of a retractor assembly, according to another exemplary embodiment of the invention;

Fig. 6 is a schematic view of a retractor assembly, according to yet another exemplary embodiment of the invention;

Fig. 7 is a schematic view of a retractor assembly, according to a further exemplary embodiment of the invention;

Fig. 8A is a perspective view of a bite block assembly for use with the guide tube of Fig. 1, according to an exemplary embodiment of the invention;

Fig. 8B is a plan view of the exemplary bite block assembly of Fig. 8A;

Fig. 9A is a schematic view of a bite block assembly and a guide tube inserted into a patient, according to an exemplary embodiment of the invention;

Fig. 9B is a cross-sectional view of the exemplary bite block assembly and guide tube of Fig. 9A;

Fig. 10 is a schematic view of a bite block assembly for use with a guide tube inserted into a patent, according to another exemplary embodiment of the invention;

Fig. 11 is a perspective view of a bite block assembly for use with a guide tube, according to yet another exemplary embodiment of the invention;

Fig. 12 is a schematic view of a bite block assembly for use with a guide tube inserted into a patient, according to a further exemplary embodiment of the invention;

Fig. 13A is a cross-sectional view of a bite block assembly, according to a further exemplary embodiment of the invention;

Fig. 13B is a rear view of the exemplary bite block assembly of Fig. 13A;

Fig. 13C is a perspective view of a guide tube for coupling with the exemplary bite block assembly of Fig. 13A;

Fig. 14A is a cross-sectional view of an upper portion of a bite block assembly, according to a further exemplary embodiment of the invention;

Fig. 14B is a cross-sectional view of a lower portion of the exemplary bite block assembly of Fig. 14A;

Fig. 14C is a rear cross-sectional view of the exemplary bite block assembly of Fig. 14A; and

Fig. 14D is a perspective view of a guide tube for coupling with the exemplary bite block of Fig. 14A.

### DESCRIPTION OF THE EMBODIMENTS

Reference will now be made in detail to exemplary embodiments of the invention, examples of which are illustrated in the accompanying drawings. Wherever possible, the same reference numbers will be used throughout the drawings to refer to the same or like parts.

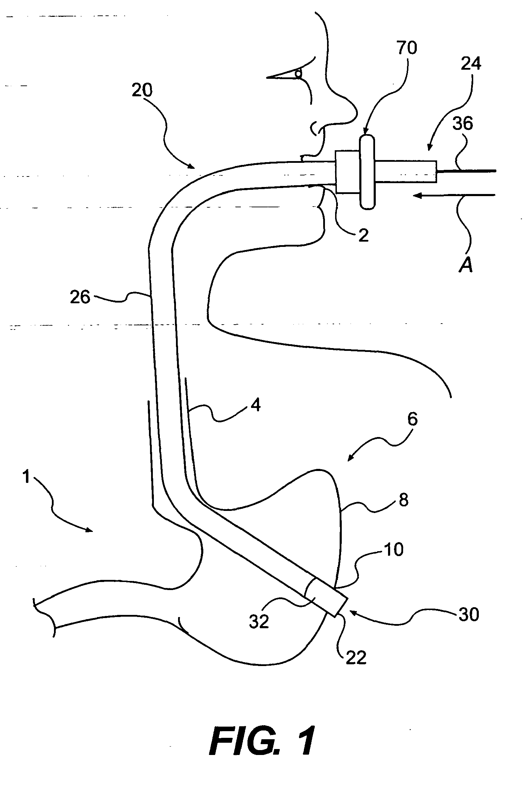

Figs. 1-3 depict an exemplary guide tube 20 that is capable of being used for the purpose of performing transluminal endoscopic surgery. The guide tube 20 may be configured to be advanced through any body lumen, e.g., a gastrointestinal (GI) tract 1 including a mouth 2, an esophagus 4, and a stomach 6, of a patient. The guide tube 20 may have a distal end 22 and a proximal end 24, and may be made of any suitable material, e.g., a suitable biocompatible material capable of being advanced through the GI tract 1. The guide tube 20 may include an elongated member 26 extending between the distal end 22 and the proximal end 24. The elongated member 26 may be flexible, for example, to be able to traverse tortuous anatomy. In the configuration depicted in Figs. 1-3, the distal end 22 may include the-end of the guide tube 20 internal to the body and the proximal end 24 may include the end of the guide tube 20 external to the body.

According to an embodiment, the guide tube 20 may be inserted through the mouth 2 or other body orifice (e.g., anus, vagina, nose, percutaneous incision,etc.). The guide tube 20-may be advanced through the esophagus 4 and then inserted into the stomach 6 of the patient. The guide tube 20 may include a device (not shown) for making an incision 10 through a wall 8 of the stomach 6 or other body tissue, such as a wall of another body organ. The guide tube 20 may then pass through the incision 10 to operate at a work site. The work site could include, for instance, a part of the small intestine (not shown), another organ within the abdominal cavity, or other body organ. It should be emphasized that the illustrated application of the guide tube 20 is exemplary only and that the inventions of the current disclosure may be applied to any endoscopic surgical application known in the art. For example, the guide tubes described herein may be inserted into a body cavity instead of, or in addition to, being advanced through a body lumen, e.g., percutaneously, through an incision in the skin.

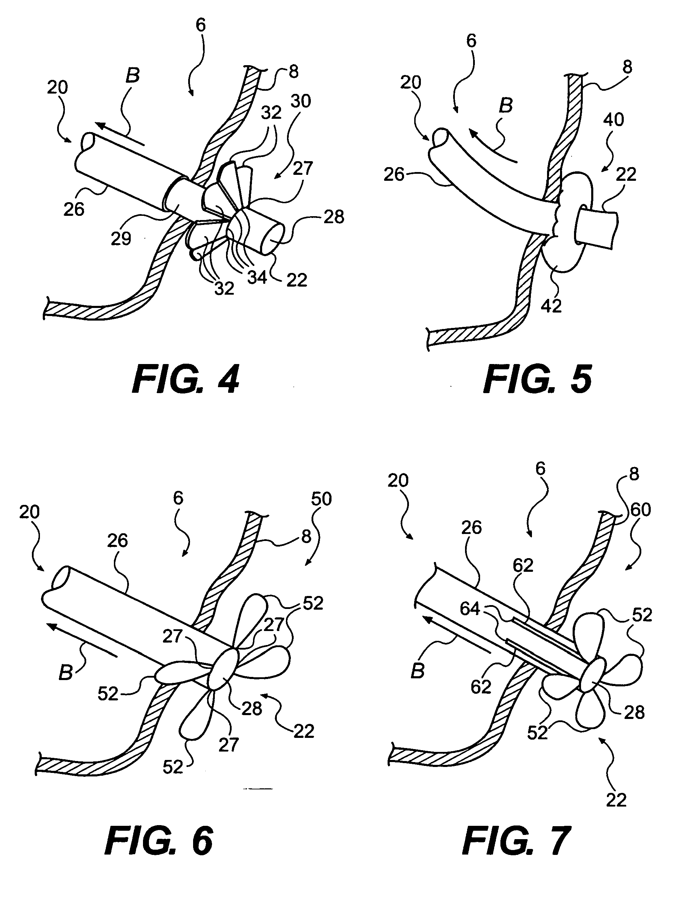

A lumen 28 (Fig. 4) may run longitudinally through the elongated member 26 of the guide tube 20 and may include a hollow cavity configured to deliver an endoscopic instrument (not shown) to the work site. The lumen 28 may extend between the distal end 22 and the proximal end 24 of the guide tube 20. Alternatively, one or more additional lumens (not shown), e.g., one or more of an aspiration lumen, an irrigation lumen, an illumination lumen, a viewing lumen, and a working lumen, may run longitudinally through the elongated member 26.

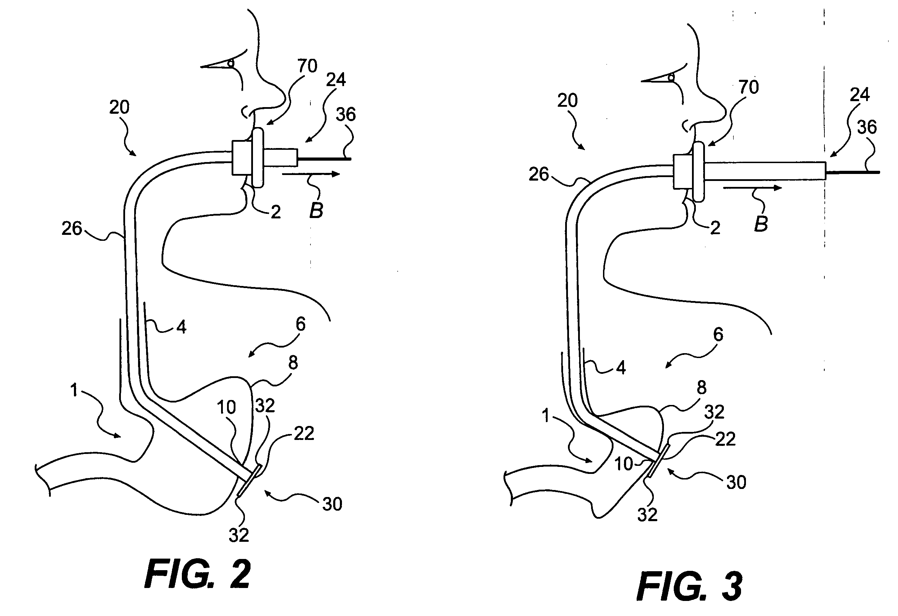

A retractor assembly 30 may be disposed on the elongated member 26 of the guide tube 20 on or near the distal end 22. The retractor assembly 30 may be attached to the distal end 22 of the elongated member 26 using any suitable method and/or mechanism. Alternatively, the retractor assembly 30 may be capable of moving longitudinally with respect to the elongated member 26, as described below. The retractor assembly 30 is configured to be in a collapsed (unexpanded) configuration (Fig. 1) or an expanded configuration (Figs. 2 and 3). In the collapsed configuration, the retractor assembly 30 lies substantially flat against an outer surface of the elongated member 26 or can be stowed within the lumen 28 of the guide tube 20. In the expanded configuration, the retractor assembly 30 extends radially from the outer surface of the elongated member 26 with respect to an axis of the elongated member 26. The retractor assembly 30 may be made out of any suitable material to allow it to be moved between the collapsed and expanded configurations.

One or more linkages 36, e.g., tubes,cables, wires, etc., may be disposed within the lumen 28 and may extend through one or more openings 27 in the elongated member 26 to connect to the retractor assembly 30. Alternatively, the retractor assembly 30 may extend through the opening 27 to connect to the linkage 36. The retractor assembly 30 may be-connected via the linkage 36 to an actuating mechanism (not shown) near the proximal end 24 of the guide tube 20. The linkage 36 may cause the retractor assembly 30 to move between the extended and collapsed configurations, as described below. The linkage 36 may also cause the retractor assembly 30 to move longitudinally with respect to the elongated member 26, as described below.

The retractor assembly 30 may be in the collapsed configuration when the distal end 22 of the guide tube 20, on which the collapsed retractor assembly 30 is disposed, is inserted in the patient's mouth 2 and advanced through the esophagus 4 and into the stomach 6 in the direction of arrow A. After the incision 10 is made in the stomach wall 8, the distal end 22 of the guide tube 20 is advanced through the incision 10. According to the invention the elongated member 26 Ynay be secured in place by a stabilizing assembly, such as a bite block assembly 70, disposed about the guide tube 20 on or near the proximal end 24, as discussed below.

When the retractor assembly 30 is outside the stomach 6 and within the abdominal cavity, the retractor assembly 30 may be expanded from the collapsed configuration to the expanded configuration. In the expanded configuration, the retractor assembly 30 may act as a gastric seal or other type of seal for at least partially or completely occluding the incision 10 around the elongated member 26. Then, the elongated member 26 may be pulled in the direction of arrow B. In the expanded configuration, the retractor assembly 30 may move at least a portion of the stomach wall 8 as the elongated member 26 is pulled in the direction of arrow B. As shown in Fig. 3, the stomach 6 may deflate and the stomach wall 8 may be retracted to create more space in the work site.

Alternatively, or in addition, the retractor assembly 30 may retract other body tissue other than the body tissue through which the guide tube 20 passes and/or may deflate or retract other body organs other than the body organ through which the guide tube 20 passes to create more space in the work site. The retractor-assembly 30 may retract the other body tissue or body organ near the distal end of the guide tube 20, e.g., near an exit opening of the lumen 28 at the distal end of the guide tube 20. As another alternative, or in addition, the retractor assembly 30 may extend outward in front of the guide tube 20, i.e., in front of the distal end 22 of the guide tube 20, to push-or-move the other body tissue or body organ to be retracted. As a result, the retractor assembly 30 may retract (or push against, apply a force to, move, etc.) a body organ, a wall of a body organ, a body lumen, or any other type of body tissue through longitudinal displacement of the elongated member 26, and may create a working volume for using the working instruments. The retractor assembly 30 is capable of moving with respect to a reference point, e.g., the location of the bite block assembly 70 (or other stabilizing assembly at the point of entry into the patient's body).

With increased space at the work site, the working instruments delivered through the lumen 28 to the distal end 22 of the guide tube 20 may have more space to maneuver. Furthermore, viewing instruments (e.g., a camera or other device) used to deliver an image of the work site external to the patient's body may also have more space to maneuver, thereby improving visualization at the work site.

According to an exemplary embodiment, the retractor assembly 30 may be a flared flange retractor that may include one or more tabs or flanges 32. As shown in Fig. 4, the retractor assembly 30 may include multiple flanges 32 that surround the circumference of the elongated member 26 near the distal end 22. The linkage 36 may connect to the multiple flanges 32 and may extend longitudinally within the lumen 28 toward the proximal end 24 of the guide tube 20.

In Fig. 1, the flanges 32 are shown in a collapsed configuration, i.e., the flanges 32 are positioned at approximately 0° with respect to the surface of the elongated member 26, thereby resulting in the collapsed configuration of the retractor assembly 30 as described above. In other words, the flanges 32 are positioned so that they overlap the outer surface of the elongated member 26. Alternatively, as shown in Fig. 4, the outer surface of the elongated member 26 may include a groove 29, and when the retractor assembly 30 is in the collapsed configuration, the flanges 32 may be disposed within the groove 29 so that an outer surface of each flange 32 is flush against the outer surface of the elongated member 26 surrounding the groove 29. In another alternative, in the collapsed configuration, the retractor assembly 30 may have a larger diameter (or other dimension such as width) than the diameter of the elongated member 26. For example, the retractor assembly 30 in the collapsed configuration may have a conical shape (e.g., the partially flared configuration described below) that allows dilatation of the incision 10 in-the stomach wall 8 when the retractor assembly 30 is advanced through the incision 10.

In Fig. 4, the flanges 32 are shown in a partially flared configuration, i.e., the flanges 32 are positioned at an angle less than 90° and greater than 0° with respect to the outer surface of the elongated member 26. The flanges 32 may be -positioned in this partially flared configuration when the retractor assembly 30 is in the expanded configuration. In Figs. 2 and 3, the flanges 32 are shown in a fully flared configuration, i.e., the flanges 32 are positioned at an approximately 90° angle with respect to the outer surface of the elongated member 26, which also results in the expanded configuration of the retractor assembly 30. Thus, the flanges 32 may expand to the partially or fully flared configuration when the retractor assembly 30 is set to the expanded configuration.

According to an embodiment, the linkage 36 may be controlled to cause the flanges 32 to flare outward to the expanded configuration. For example, the linkage 36 may be connected to an inner edge 34 of each flange 32 such that when the linkage 36 is pulled in the direction of arrow B via the actuation mechanism, the inner edge 34 of each flange 32 slides toward the opening 27 in the elongated member 26, causing each flange 32 to tilt and move toward the expanded configuration. The stomach 6 may be deflated and/or the stomach wall 8 may be retracted by the partially or fully flared flanges 32 by pulling the elongated member 26 in the direction of arrow B. Then, before removing the guide tube 20 from the GI tract 1, the flanges 32 may be returned to the collapsed configuration via the linkage 36. The linkage 36 may include a spring mechanism or other biasing mechanism for allowing the flanges 32 to return to the collapsed configuration when the pulling force transmitted via the actuation mechanism in the direction of arrow B is released.

The retractor assembly 30 may also include any suitable shape, parts, and/or configurations to accomplish the objectives set forth herein, e.g., moving between the collapsed and expanded configurations and allowing the retraction of one or more walls of body organs or other body tissue at the work site. For example, Figs. 5-7 illustrate alternate embodiments of the retractor assembly 30 of Figs. 1-4.

In the exemplary embodiment shown in Fig. 5, a retractor assembly 40 may be a balloon retractor that may include an annular or donut-shaped balloon 42 surrounding a circumference of the elongated member 26. The balloon 42 may be disposed on the elongated member 26 of the guide tube 20 near the distal end 22. The balloon 42 may be connected via the linkage 36 (e.g., a tube) to a fluid source (not shown) so that the balloon 42 may be inflated or deflated.

Before the guide tube 20 is inserted into the patient's GI tract 1, the balloon 42 may be deflated by allowing air or another fluid (e.g., another gaseous fluid, water, or other liquid) to escape through the linkage 36. When the balloon 42 is deflated, the retractor assembly 40 is in the collapsed configuration. After the collapsed retractor assembly 40 is advanced in the GI tract 1 in the direction of arrow A (Fig. 1) and passed through the incision 10 in the stomach wall 8, the balloon 42 may be inflated by supplying air through the linkage 36 to the balloon 42 to set the retractor assembly 40 in the expanded configuration, which is shown in Fig. 5. Then, the stomach 6 may be deflated and/or the stomach wall 8 may be retracted by the inflated balloon 42 by pulling the elongated member 26 in the direction of arrow B. Before removing the guide tube 20 from the GI tract 1, the balloon 42 may be deflated by removing the air from the balloon 42 via the linkage 36 so that the retractor assembly 40 is in the collapsed configuration. Alternatively, inflating the balloon 42 allows the balloon 42 to apply a radial force against a body organ, a wall of a body organ, a body lumen, or any other type of body tissue. For example, as the balloon 42 is inflated inside a body lumen or body cavity, the balloon 42 may apply an outward radial force on an inner surface of the body lumen or body cavity causing the body lumen or body cavity to expand radially. As a result, the retractor assembly 40 may retract (or push against, apply a force to, move, etc.) a body organ, a wall of a body organ, a body lumen, or any other type of body tissue through radial expansion of the retractor assembly 40, e.g., the balloon 42, and/or through longitudinal displacement of the elongated member 26. The retractor assembly 40 is capable of moving with respect to a reference point, e.g., the location of the bite block assembly 70 (or other stabilizing assembly at the point of entry into the patient's body).

In the exemplary embodiment shown in Fig. 6, a retractor assembly -50-may be a loop retractor that may include one or more loops 52 (e.g., four loops) disposed on the elongated member 26 of the guide tube 20 on the distal end 22. The loops 52 may be formed of wire, cable, or other strand of rigid or semi-rigid material. Movement of the linkage 36 in the direction of arrow B may cause the loops 52 to expand outward to the expanded configuration, i.e., the loops 52 are positioned at an approximately 90° angle with respect to the outer surface of the elongated member 26. The linkage 36 may be connected to each of the loops 52. When the linkage 36 is pulled in the direction of arrow B, each loop 52 slides toward the opening 27 in the elongated member 26, causing the loops 32 to tilt and move toward the expanded configuration. When the pulling force transmitted via the actuation mechanism in the direction of arrow B is released, the loops 52 may be free to move back into the collapsed configuration, i.e., at approximately 0° with respect to the outer surface of the elongated member 26.

Before the guide tube 20 is inserted into the patient's GI tract 1, the loops 52 may be in the collapsed configuration so that the retractor assembly 50 is in the collapsed configuration. For example, in the collapsed configuration, the loops 52 may overlap or may lie flat against the outer surface of the elongated member 26 or may be pulled into the elongated member 26 so that the retractor assembly 50 is in the collapsed configuration. After the collapsed retractor assembly 50 is advanced in the GI tract 1 in the direction of arrow A (Fig. 1) and passed through the incision 10 in the stomach wall 8, the loops 52 may be set in the expanded configuration, which is shown in Fig. 6. Then, the stomach 6 may be deflated and/or the stomach wall 8 may be retracted by the loops 52 by pulling the -elongated member 26 in the direction of arrow B while the loops 52 are in the expanded configuration. Before removing the guide tube 20 from the GI tract 1, the 100ps 52 may be returned to the collapsed configurations as described above. Alternatively, moving the loops 52 to the expanded configuration allows the loops 52 to apply a radial force against a body organ, a wall of a body organ, a body lumen; or any other type of body tissue. For example, as the loops 52 are expanded inside a bod lumen or body cavity,the loops 52 may apply an outward radial force-on an inner surface of the body lumen or body cavity causing the body lumen or body cavity to expand radially. As a result, the retractor assembly 50 may retract (or push against, apply a force to, move, etc.) a body organ, a wall of a body organ,a body lumen, or any other type of body tissue through radial expansion of the retractor assembly 50, e.g., the loops 52, and/or through longitudinal displacement of the elongated member 26. The retractor assembly 50 is capable of moving with respect to a reference point, e.g., the location of the bite block assembly 70 (or other stabilizing assembly at the point of entry into the patient's body).

In the exemplary embodiment shown in Fig. 7, a retractor assembly 60 may be similar to the retractor assembly 50 shown in Fig. 6, which includes the loops 52, except that the elongated member 26 may include one or more slots 62 that each extend from the distal end 22 of the elongated member 26 to an intermediate location 64 between the distal and proximate ends 22, 24 of the elongated member 26. When the retractor assembly 60 is in the expanded configuration, as shown in Fig. 7, the loops 52 may be guided by the slots 62 to slide longitudinally in the direction of arrow B. The retractor assembly 60 may be retracted by sliding the loops 52 within the slots 62 to the intermediate locations 64 in the direction of arrow B. In addition, like the retractor assembly 50 shown in Fig. 6, the elongated member 26 may be pulled in the direction of arrow B to retract the retractor assembly 60. Accordingly, the stomach 6 may be deflated and/or the stomach wall 8 may be retracted by the loops 52 as the loops 52 move within the slots 62 from the distal end 22 of the guide tube 20 to the intermediate locations 64 and/or by pulling the elongated member 26 in the direction of narrow B while the loops 52 are in the expanded configuration. Alternatively, as described above, moving the loops 52 to the expanded configuration allows the loops 52 to apply a radial force-against a body organ, a wall of a body organ, a body lumen, or any other type of body tissue. As a result, the retractor assembly 60 may retract (or push against, apply a force to, move, etc.) a body organ, a wall of a body organ, a body lumen, or any other type of body tissue through radial expansion of the retractor assembly 60, e.g., the loops 52, through the longitudinal displacement of the retractor assembly 60, e.g., the loops 52, and/or through longitudinal displacement of the elongated member 26. The retractor assembly 60 is capable of moving with respect to a reference point, e.g., the location of the bite block assembly 70 (or other stabilizing assembly at the point of entry into the patient's body).

Although the embodiments described below refer to the retraction assembly 30, it is to be understood that the embodiments described below may include any type of retraction assembly described above, e.g., the retraction assemblies 40, 50, 60 shown in Figs. 5-7.

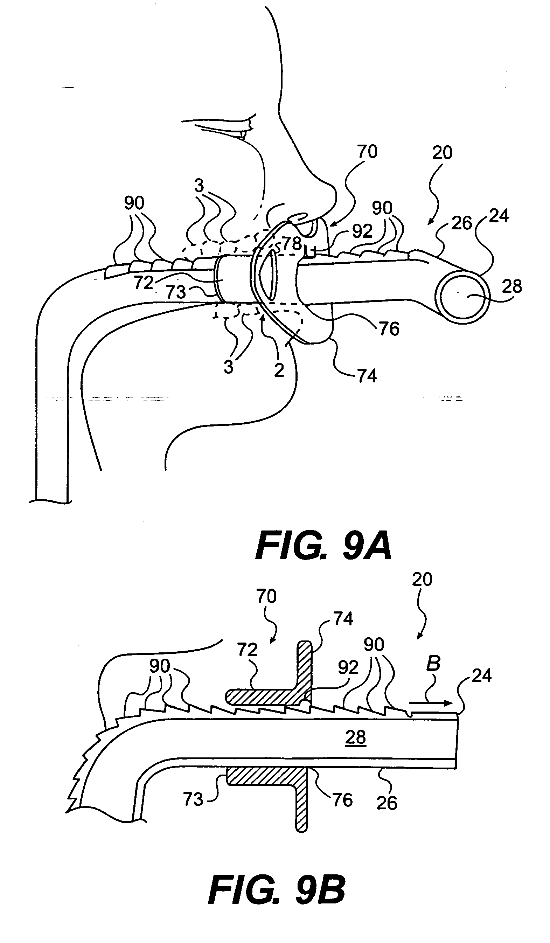

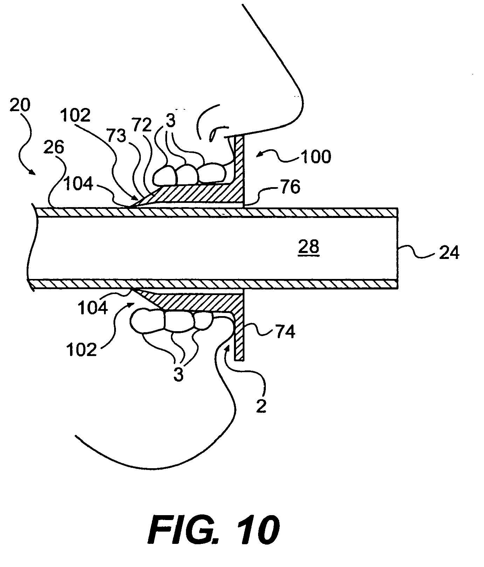

As shown in Figs. 1-3, the bite block assembly 70 may be disposed about the guide tube 20 on or near the proximal end 24. The bite block assembly 70 is configured to allow the patient to bite down on the bite block assembly 70 to allow the patient to grip the bite block assembly 70 between opposed teeth 3 (Figs. 9A and 10) of the upper and lower jaws in the mouth 2. The bite block assembly 70 may be made of any suitable material, e.g., a suitable biocompatible material capable of being bitten and secured between the patient's jaws, such as rubber or plastic.

Alternatively, instead of inserting the guide tube 20 and the bite block assembly 70 in the patient's mouth 2, the guide tube 20 and a stabilizing assembly generally similar to the bite block assembly 70 may be inserted into other points of entry into the patient's body, such as the anus, vagina, nose, or other orifice. For example, an entry clamp may be used, when appropriate, to lock the guide tube 20 with respect to the orifice. In an exemplary embodiment, a stabilizing assembly may be inserted into the patient's anus, vagina, nose, or other orifice, and may be securable to, for example, the patient's leg, head, or other body part. In an exemplary embodiment, a stabilizing assembly, such as a percutaneous port or a percutaneous trocar, may be inserted into an incision in the skin and may be securable to, for example, a portion of the patient's body tissue or a body part. It is to be understood that elements of the embodiments of the bite block assemblies described herein may also be included in stabilizing assemblies inserted into the patient's anus vagina, nose, incision in the skin, or other orifice.

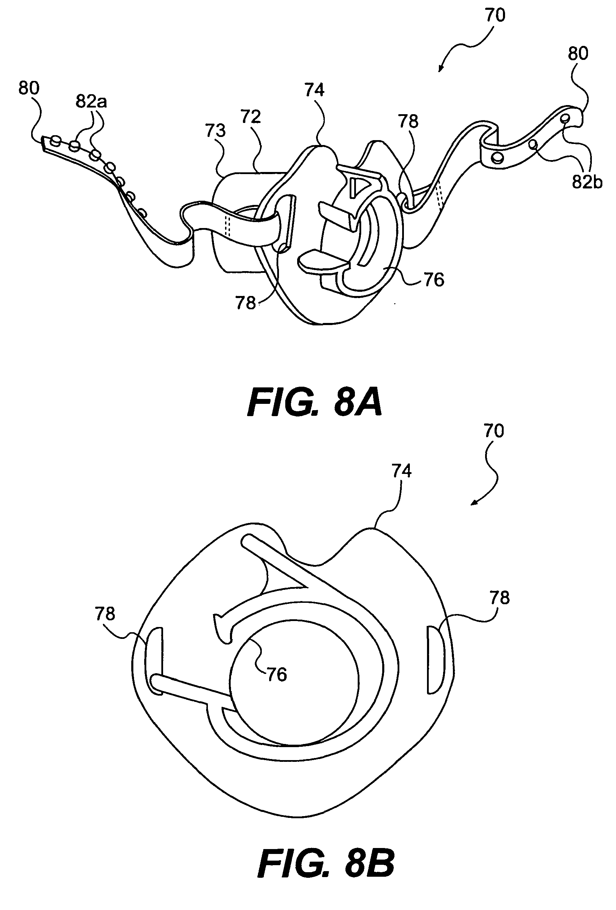

As shown in Figs. 8A and 8B, the bite block assembly 70 includes a tubular mouthpiece 72 and a front shield 74. The mouthpiece 72 may be formed integrally with the shield 74. A passageway 76 is formed by an inner surface of the mouthpiece 72 and the shield 74, and the elongated member 26 may be slidably disposed in the passageway 76. The shield 74 also includes a pair of strap openings 78. One or more straps 80 may be attached to the strap openings 78 in the shield 74. As shown in Fig. 8A, a pair of straps 80 are connected to the strap openings 78 and wrap around the back of the patient's head to secure the bite block assembly 70 in place. The straps 80 are provided with a fastening mechanism 82 that is adjustable to vary the fit of the straps 80 around the head. For example, the fastening mechanism 82 may include a plurality of locking nubs 82a on one strap 80 and holes 82b on the other strap 80. After securing the bite block assembly 70 to the patient, the elongated member 26 may be inserted into the bite block assembly 70 and advanced into the patient's GI tract 1. Then, the retractor mechanism 30 may be operated as described above.

Alternatively, the elongated member 26 may be capable of being locked in place to the bite block assembly 70 to prevent inadvertent movement of the elongated member 26. The bite block assembly 70 may include any suitable shape, parts, and/or configurations to allow the bite block assembly 70 to be secured or locked to the elongated member 26. For example, Figs. 9A, 9B, 10-12, 13A-13C, and 14A-14D illustrate alternate embodiments of the bite block assembly 70 and/or the elongated member 26 of the guide tube 20 of Figs. 1-3, 8A, and 8B in which the bite block assembly is a location locking bite block assembly that releasably locks the bite block assembly 70 to the elongated member 26.

With a location locking bite block assembly, the location of the bite block assembly 70 on the guide tube 20 and the positioning of the guide tube 20 in the body may be adjusted to position the working instruments within the body. The guide tube 20 may be used to deliver the working instruments through the lumen 28 so that the working instruments may be used to operate inside the body. The bite block assembly 70 may be used as a frame of reference so that the adjustment of the guide tube 20, e.g., by moving in the direction of arrow A (Fig. 1) or arrow B (Figs. 2 and 3) with respect to the bite block assembly 70, allows the lengthening or shortening of the amount of the guide tube 20 in the body. -For example, the guide tube 20 may be inserted into the patient's GI tract 1 in the direction of arrow A until the distal end 22 of the guide tube 20 is positioned, e.g., at a floor of the stomach 6. Then, the bite block assembly 70 may be locked onto the elongated member 26 of the guide tube 20. The working instruments (e.g., an endoscope or other instrument including the device for making the incision 10) may be inserted through the guide tube 20 and may create the incision 10 in the stomach wall 8. Then, the bite block assembly 70 may be released or unlocked from the elongated member 26 of the guide tube 20. The guide tube 20 may be advanced further into the GI tract 1 in the direction of arrow A so that the distal end 22 of the guide tube 20 is advanced through the incision 10 in the stomach wall 8, e.g., toward another organ such as a gall bladder. Then, the bite block assembly 70 is locked onto the elongated member 26 of the guide tube 20 for a second time. The working instruments may be manipulated through the guide tube 20 to operate on the gall bladder (e.g., during a cholecystectomy) or other organ. As a result, the bite block assembly 70 may be used as a frame of reference when inserting the guide tube 20 and the working instruments into the body.

Alternatively, before operating on the gall bladder or other organ, the retractor assembly 30 may be expanded, and the bite block assembly 70 may be released or unlocked from the elongated member 26 of the guide tube 20 again. Then, the guide tube 20 may be pulled in the direction of arrow B so that the retractor assembly 30 may deflate the stomach 6 and retract the stomach wall 8 to create more space in the work site. Then, the bite block assembly 70 may be locked onto the elongated member 26 of the guide tube 20 for a third time before manipulating the working instruments through the guide tube 20 to operate on the gall bladder or other organ.

In the exemplary embodiment shown in Figs. 9A and 9B, the bite block assembly 70 may be a location locking bite block assembly that allows the elongated member 26 to slide therethrough and is also capable of being secured onto the elongated member 26. A series of ratchet teeth 90 may be integrally formed along the outer surface of the elongated member 26 in a line extending in the longitudinal direction. The rachet teeth 90 may be located at incremental locations along the outer surface of the elongated member 26. A notch 92 is formed in the passageway 76 in the shield 74 of the bite block assembly 70, and the notch 92 opposes the series of rachet teeth 90 when the elongated member 26 is inserted into the passageway 76. Alternatively, the notch 92 may be formed in the passageway 76 in the mouth piece 72. As another alternative, the notch 92 may instead be formed-on the outer surface of the elongated member 26, and the series of ratchet teeth 90 may be integrally formed inside the bite block assembly 70.

After the elongated member 26 is inserted into the patient's GI tract 1, the bite block assembly 70 may be positioned on the elongated member 26 and secured to the patient prior to the insertion of any working instruments into the elongated member 26. In an embodiment, the retractor mechanism 30 may be expanded to the expanded configuration, and the elongated member 26 may be pulled back in the direction of arrow B (Figs. 2 and 3). Since the retractor mechanism 30 is in the expanded configuration, the retractor mechanism 30 may deflate the stomach 6 and/or retract the stomach wall 8. Also, as the elongated member 26 is pulled back in the direction of arrow B, the rachet teeth 90 slide against the surface of the passageway 76, as shown in Fig. 9B, until one of the rachet teeth 90 engages with the notch 92. When the rachet tooth 90 engages with the notch 92, the elongated member 26 and the bite block assembly 70 are locked together until the elongated member 26 is moved again in the direction of arrow B with respect to the bite block assembly 70. The rachet teeth 90 are formed such that the elongated member 26 may move in the direction of arrow B with respect to the bite block assembly 70, but the elongated member 26 is prevented from moving in the opposite direction.

The elongated member 26 may be pulled in the direction of arrow B to adjust the position of the elongated member 26 to the bite block assembly 70, thereby allowing customization based on-the dimensions of the individual patient's anatomy (e.g., the distance between the mouth 2 and the stomach 6). Furthermore, the- bite block assembly 70 releasably locks the elongated member 26 in place as each rachet tooth 90 engages with the notch 92. Accordingly, the elongated member 26 is prevented from advancing back into the patient's GI tract 1. The operator does not have to continuously hold the elongated member 26 to secure the elongated member 26 with respect to the bite block assembly 70 since the bite block assembly 70 may be secured at various predetermined and incremental positions along the elongated member 26. Therefore, inadvertent movement of the elongated member 26, e.g., during surgery, may be prevented.

In the exemplary embodiment shown in Fig. 10, a bite block assembly 100 may be another location locking bite block assembly. The bite block assembly 100 may be similar to the bite block assembly 70 shown in Figs. 9A and 9B except that a friction fit may be formed between the elongated member 26 and the bite block assembly 100. For example, a seal 102 or other member configured to form a friction fit between the elongated member 26 and the bite block assembly 100 may be disposed on a distal end 73 of the mouthpiece 72. The seal 102 may be formed integrally with or separately from the mouthpiece 72 and the shield 74. The seal 102 may be made of the same or different material as the mouthpiece 72 and/or the shield 74, such as the materials described above.

The seal 102 may be annular and may decrease in diameter toward a distal end 104 of the seal 102. The diameter at the distal end 104 of the seal 102 may be approximately equal to, or slightly less than, a diameter of the outer surface of the elongated member 26. Accordingly, the distal end 104 of the seal 102 may contact the outer surface of the elongated member 26 when the elongated member 26 is disposed within the passageway 76 of the bite block assembly 100.

After securing the bite block assembly 100 to the patient, the elongated member 26 is inserted into the bite block assembly 100 and advanced into the patient's GI tract 1. Then, the retractor mechanism 30 may be operated as described above. The position of the elongated member 26 with respect to the bite block assembly 100 is freely adjustable, and the seal 102 forms a friction fit between the elongated member 26 and the bite block assembly 100 to releasably hold the bite block assembly 100 on the elongated member 26. The operator does not have to continuously hold the elongated member 26 to secure the elongated member 26 with respect to the bite block assembly 100 since the bite block assembly 100 maybe secured at any position along the elongated member 26 selected by the operator. Thus, inadvertent movement of the elongated member 26, e.g., during surgery, may be prevented.

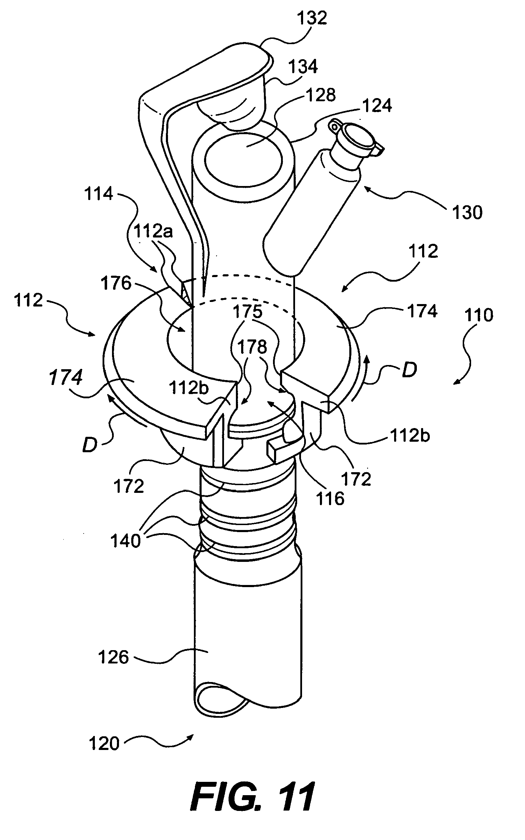

In the exemplary embodiment shown in Fig. 11, a guide tube 120 is shown in combination with a bite block assembly 110, which may be a location locking bite block assembly that includes two hinged-portions 112. The hinged portions 112 are capable of surrounding a portion of the circumference of an elongated member 126 of the guide tube 120. The hinged portions 112 include first sides 112a and second sides 112b that extend longitudinally and radially. The first sides 112a of the respective hinged portions 112 oppose each other and are connected by a hinge 114. The second sides 112b of the respective hinged portions 112 oppose each other and are separated by a gap 116. The hinge 114 allows the second sides 112b to move away from each other in the directions of arrow D such that the bite block-assembly 110 is in an open configuration. The second sides 112b may be moved toward each other in the opposite direction to set the bite block assembly 110 in a closed configuration.

Each of the hinged portions 112 includes a mouthpiece portion 172 and a front shield portion 174 such that when the bite block assembly 110 is in the closed configuration, the patient may bite down on the mouthpiece portions 172, as described above in connection with the mouthpiece 72 of the bite block assembly 70 shown in Figs. 8A and 8B. The shield portions 174 may include a pair of strap openings (e.g., strap openings 78 shown in Figs. 8A and 8B). The hinged portions 112 may include a passageway 176 formed by an inner surface 175 of the mouthpiece portions 172 and the shield 174. Furthermore, a cavity 178 may be formed in the inner surface 175 of the hinged portions 112 extending between the first and second sides 112a, 112b.

The elongated member 126 of the guide tube 120 may be slidably disposed in the passageway 176 formed by the hinged portions 112. A radius of the inner surface 175 of the hinged portions 112 may be approximately equal to a radius of an outer surface of the elongated member 126. The elongated member 126 may be similar to the elongated member 26 described above and shown in Figs. 1-7. Additionally, the guide tube 120 may be provided with a luer 130 near a proximal end 124 of the guide tube 120. An operator may administer an injection through the luer 130 to the work site using a needle (not shown). The luer 130 may be connected to a lumen 128 that runs longitudinally through the elongated member 126 or to a separate lumen. The luer 130 may include a septum or a valve. The guide tube 120 may also include a cap 132 with a stopper 134 at the proximal end 124 of the guide tube 120. The cap 132 may be closed so that the stopper 134 may be inserted into the proximal end 124 of the guide tube 120 to close a proximal opening of the lumen 128. Accordingly, when the cap 132 is closed, the needle may be used to suction fluid from the work site via the luer 130.

A series of raised rings 140 may be integrally formed along the outer surface of the elongated member 126. The raised rings 140 may be located at incremental locations along the outer surface of the elongated member 126. When the elongated member 126 is disposed in the passageway 176 and the bite block assembly 110 is in the closed configuration, as shown in Fig. 11, one of the raised rings 140 may be disposed in the cavity 178. In an alternative embodiment, the raised rings 140 may instead be integrally formed in the inner surface 175 of the hinged portions 112 extending between the first and second sides 112a, 112b, and a series of cavities 178 may be formed along the outer surface of the elongated member 126.

After the elongated member 126 is inserted into the patient's GI tract 1 and the retractor mechanism 30 is expanded to the expanded configuration, the elongated member 126 may be pulled back in the direction of arrow B (Figs. 2 and 3). To allow the elongated member 126 to move with respect to the bite block assembly 110, the bite block assembly 110 may be set in the open configuration using the hinge 114. In the open configuration, the raised rings 140 do not prevent the elongated member 126 from passing through the passageway 176. Thus, the elongated member 126 may advance in the direction of arrow B to deflate the stomach 6 and/or retract the stomach wall 8. Then, the bite block assembly 110 may releasably lock the elongated member 126 in place by setting the bite block assembly 110 in the closed configuration. In the closed configuration, one of the raised rings 140 is disposed in the cavity 178 of the hinged portions 112 of the bite block assembly 110, thereby preventing the elongated member 126 from moving in the direction of arrow B or in the reverse direction. Accordingly, the elongated member 126 is prevented from moving with-respect to the patient's GI tract 1.

Thus, the position of the bite block assembly 110 on the elongated member 126 is adjustable depending on which raised ring 140 is enclosed in the cavity 178 of the bite block assembly 110. The position of the elongated member 126 on the bite block assembly 110 may be customized based on the dimensions of the individual patient's anatomy (e.g., the distance between the mouth 2 and the stomach 6). Furthermore, the operator does not have to continuously hold the elongated member 126 to keep the elongated member 126 from moving with respect to the bite block assembly 110 since the bite block assembly 110 is configured to be secured at various predetermined and incremental positions along the elongated member 126. Inadvertent movement of the elongated member 126, e.g., during surgery, may be prevented.

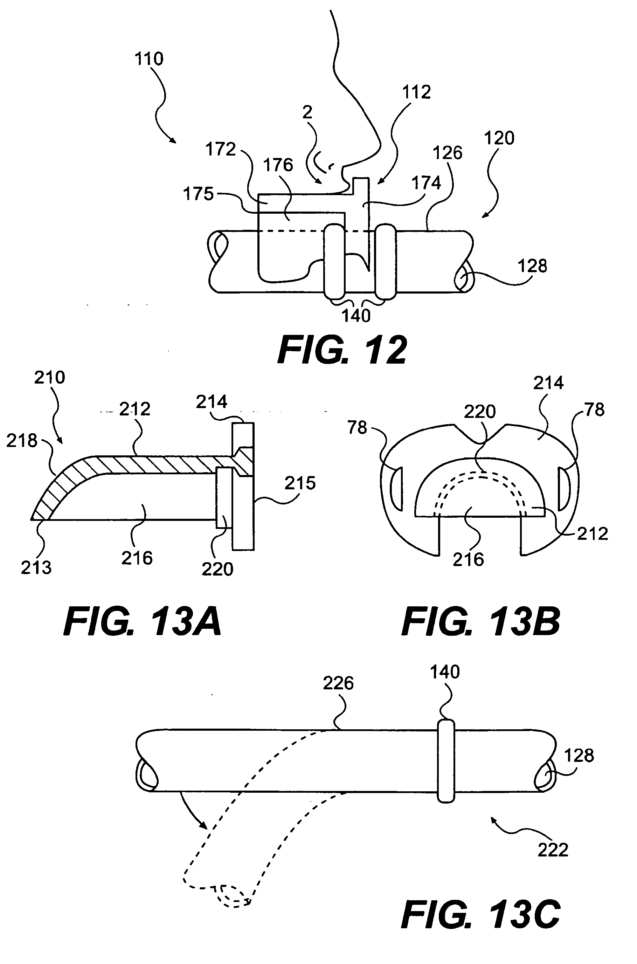

In the exemplary embodiment shown in Fig. 12, the bite block assembly 1-1-0-may-be-similar to the bite block assembly 100 shown- in Fig. 11 except that the cavity 178 in the inner surface 175 of the hinged portions 112 may be omitted and a radius of the inner surface 175 of the hinged portions 112 may be approximately equal to a radius of an outer surface of the raised rings 140. Furthermore, two raised rings 140 may be integrally formed on the outer surface of the elongated member 126.

Alternatively, a raised ring (not shown) may instead be integrally formed on the inner surface 175 of the hinged portions 112, and one or more raised rings 140 may be integrally formed on the outer surface of the elongated member 126. Therefore, one of the raised rings 140 on the elongated member 126 may be disposed between a portion of the shield 174 and the raised ring on the inner surface 175 of the hinged portions 112 when the bite block assembly 110 is set in the closed configuration. In yet another embodiment, two raised rings (not shown) may instead be integrally formed on the inner surface 175 of the hinged portions 112, and one or more raised rings 140 may be integrally formed on the outer surface of the elongated member 126. Therefore, one of the raised rings 140 on the elongated member 126 may be disposed between the two raised rings 140 on the inner surface 175 of the hinged portions 112 when the bite block assembly 110 is set in the closed configuration.

Like the embodiment shown in Fig. 11, the bite block assembly 110 may also releasably lock the elongated member 126 in place by setting the bite block assembly 110 in the closed configuration. However, in the embodiment shown in Fig. 12, in the closed configuration, a portion of the shield 174 is disposed between the two raised rings 140, thereby preventing the elongated member 126 from moving in the direction of arrow B or in the reverse direction. Accordingly, the elongated-member 126 is prevented from moving with respect to the patient's GI tract 1. The operator does not have to continuously hold the elongated member 126 to keep the elongated member 126 from moving with respect to the bite block assembly 110. Inadvertent movement of the elongated member 126, e.g., during surgery, may be prevented.

In the exemplary embodiment shown in Figs. 13A and 13B, a bite block assembly 210 may be another location locking bite block assembly that can be secured to an elongated member 226 of a guide tube 222. Fig. 13C shows the -elongated-member 226, which may be similar to the elongated member 126 described above in connection with Fig. 11, except the elongated member 226 includes a single raised ring 140.

The bite block assembly 210 includes a mouthpiece 212 and a front shield 214. The patient may bite down on the mouthpiece 212, as described above in connection with the mouthpiece 72 of the bite block assembly 70 shown in Figs. 8A and 8B. The mouthpiece 212 has a distal end 213 and a proximal end 215, which is connected to the shield 214. As shown in Fig. 13B, the mouthpiece 212 has a semi-circular cross-section that defines a passageway 216. The passageway 216 also extends through the shield 214. The shield 214 may include strap openings 78, as described above and shown in Figs. 8A and 8B.

A curved portion 218 is provided on the upper mouthpiece 212 near its distal end 213. The curved portion 218 serves as a guide to allow the elongated member 226 to curve downward as the elongated member 226 is advanced through the passageway 216 into the GI tract 1 in the direction of arrow A (Fig. 1). The curved configuration of the flexible elongated member 226 is shown by dashed lines in Fig. 13C.

As shown in Figs. 13A and 13B, an annular groove 220 is provided in an inner surface of the proximal end 215 of the mouthpiece 212. The groove 220 may be provided adjacent to the shield 214, and is sized to allow the single raised ring 140 integrally formed on the elongated member 226 (Fig. 13C) to be disposed therein. Alternatively, the groove 220 may instead be provided on the outer surface of the elongated member 226 and sized to allow a single raised ring 140 integrally formed on the inner surface of the mouthpiece 212 to be disposed therein.

After the elongated member 226 is inserted into the patient's GI tract 1 and the retractor mechanism 30 is expanded-to-the expanded configuration, the elongated member 226 may be pulled back in the direction of arrow B (Figs. 2 and 3) within the passageway 216 of the bite block assembly 210. Thus, the elongated member 226 may advance-in the direction of arrow B to deflate the stomach 6 and/or retract the stomach wall 8. The elongated member-226-advances in the direction of arrow B until the raised ring 140 on the elongated-member 226 is inserted into the groove 220 in the mouthpiece 212, thereby releasably locking in place the bite block assembly 210 on the elongated member 226. As a result, the elongated member 226 is prevented from moving with respect to the patient's GI tract 1. The operator does not have to continuously hold the elongated member 226 to keep the elongated member 226 from moving with respect to the bite block assembly 210. Inadvertent movement of the elongated member 226, e.g., during surgery, may be prevented.

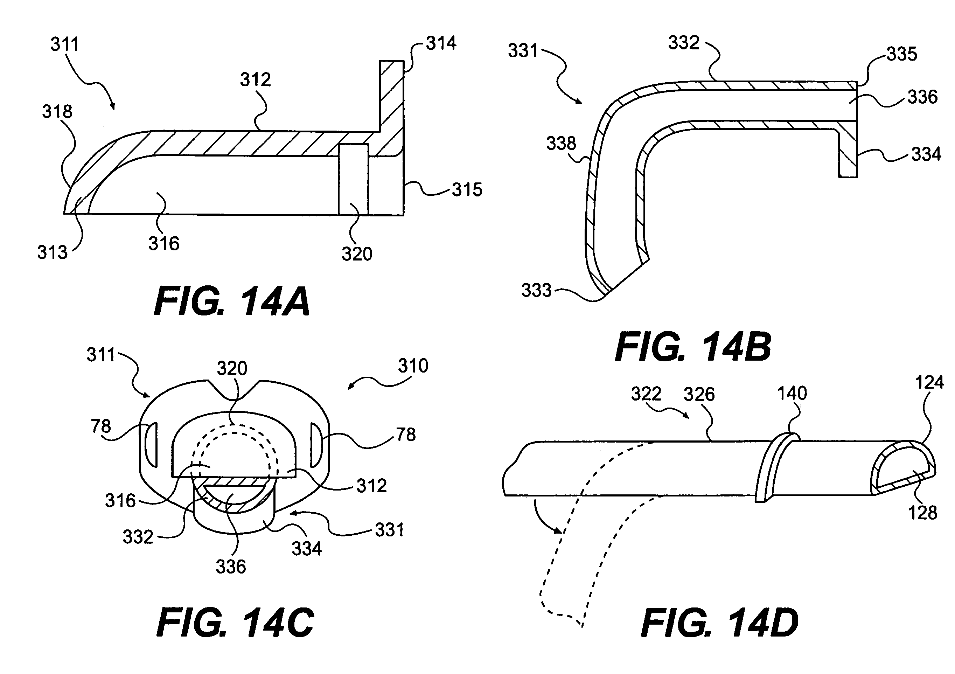

In the exemplary embodiment shown in Figs. 14A-14C, a bite block assembly 310 may be a two-component location locking bite block assembly that can lockon to an elongated member 326 of a guide tube 322. Fig. 14D shows the elongated member 326, which is similar to the elongated member 126 described above in connection with Fig. 11, except the elongated member 326 includes a single raised ring 140 and has a semi-circular cross-section.

The bite block assembly 310 includes an upper portion 311 and a lower portion 331. The upper portion 311 includes an upper mouthpiece 312 and an upper front shield 314. The lower portion 331 includes a lower mouthpiece 332 and a lower front shield 334. The upper and lower shields 314, 334 may be coplanar and may form a front shield of the bite block assembly 310. The upper mouthpiece 312 may be disposed on top of the lower mouthpiece 332, as shown in Fig. 14C, and may form a mouthpiece of the bite block assembly 310.

The patient may bite down on the upper and lower mouthpieces 312, 332, in a simitar manner as described above in connection with the mouthpiece 72 of the bite block assembly 70 shown in Figs. 8A and 8B. The upper mouthpiece 312 has a distal end 313 and a proximal end 315, which is connected to the upper shield 314. As shown in Fig. 14C, the upper mouthpiece 312 has a semi-circular cross-section that defines an upper passageway 316. The upper passageway 316 also extends through the upper shield 314, and the upper shield 314 may include strap openings 78, as described above and shown in Figs. 8A and 8B.

The lower mouthpiece 332 has a distal end 333 and a proximal end 335, which is connected to the lower shield 334. As shown in Fig. 14C, the lower mouthpiece 332 has a semi-circular cross-section that defines a lower passageway 336, which extends through the lower shield 334. The semi-circular cross-sections of the upper and lower passageways 316, 336 in the upper and lower portions 311, 331 of the bite block assembly 310 may mirror each other. The upper passageway 316 is provided for guiding the elongated member 326, as described below, and the lower passageway 336 serves as an air passage to allow the patient to breathe through the mouth 2.

A curved portion 318 is provided on the upper mouthpiece 312 near its distal end 313, and a curved portion 338 is provided on the lower mouthpiece 332 near its distal end 333. The curved portions 318, 338 serve as guides to allow the elongated member 326 to curve downward as the elongated member 326 is advanced through the passageway 316 into the GI tract 1 in the direction of arrow A (Fig. 1). The curved configuration of the flexible elongated member 326 is shown by dashed lines in Fig. 14D.

As shown in Figs. 14A and 14C, an annular groove 320 is provided in an inner surface of the proximal end 315 of the upper mouthpiece 312. The groove 320 may be provided adjacent to the upper shield 314, and is sized to allow the single raised ring 140 integrally formed on the elongated member 326 (Fig. 14D) to be disposed therein. Alternatively, the groove 320 may instead be provided on the outer surface of the elongated member 326 and sized to allow a single raised ring 140 integrally formed on the inner surface of the upper mouthpiece 312 to be disposed therein.

After the elongated member 326 is inserted into the patient's GI tract 1 and the retractor mechanism 30 is expanded to the expanded configuration, the elongated member 326 may be pulled back in the direction of arrow B (Figs. 2 and 3) within the passageway 316 of the upper portion 311 of the bite block assembly 310. As the elongated member 326 moves along the passageway 316 of the upper portion 311 of the bite block assembly 310, the elongated member 326 is also guided by an upper surface of the-lower portion 331 of the bite block assembly 310. The elongated member 326 may advance in the direction of arrow B to deflate the stomach 6 and/or retract the stomach wall 8. The elongated member 326 advances in the direction of arrow B until the raised ring 140 on the elongated member 326 is inserted into the groove 320 in the upper portion 311 of the bite block assembly 310, thereby releasably locking in place the bite block assembly 310 on the elongated member 326. As a result, the elongated member 326 is prevented from moving with respect to the patient's GI tract 1. The operator does not have to continuously hold the elongated member 326 to keep the elongated member 326 from moving with respect to the bite block assembly 310. Inadvertent movement of the elongated member 326, e.g., during surgery, may be prevented.

In the embodiments described above and shown in Figs. 11, 12, 13A-13C, and 14A-14D, the elongated member 126, 226, 326 is configured to be inserted into the bite block assembly 110, 210, 310 in a direction generally perpendicular to an axis of the elongated member 126, 226, 326. Therefore, the bite block assembly 110, 210, 310 may be secured to the patient first and then the elongated member 126, 226, 326 may be inserted into the bite block assembly 110, 210, 310, or the sequence may be reversed. In addition, the elongated member 126, 226, 326 may be inserted into the bite block assembly 110, 210, 310 even after the working instruments have been inserted into the elongated member 126, 226, 326. As a result, there is a flexibility in the order of placement of the bite block assembly 110, 210, 310, the elongated member 126, 226, 326, and the working instruments in the patient.

In various embodiments, the guide tube 20, 120, 222, 322 set forth herein may be used with any of the retractor assemblies 30, 40, 50, 60 and/or any of the bite block assemblies (stabilizing assemblies) 70, 100, 110, 210, 310 in any combination. Any aspect set forth in any embodiment may be used with any other embodiment set forth herein. Every device and apparatus set forth herein may be used in any suitable medical procedure, may be advanced through any suitable body lumen ,and body cavity, and may be used to deflate and/or retract any suitable body portion. For example, the apparatuses and methods described herein may be -used in any natural body lumen or tract, including those accessed orally, vaginally, or rectally.

The stabilizing assembly, such as the bite block assemblies 70, 100, 110, 210, 310 described herein, may serve as a movable frame of reference, and is capable of stabilizing the guide tube 20, 120, 222, 322. As a result, the distal and proximal ends of the guide tube 20,120, 222, 322 may be stabilized so that the movement of working instruments inserted through the guide tube-20, 120, 222, 322 may be controlled. Working instruments may be moved translationally without disrupting the positioning of the guide tube 20, 120, 222, 322 with respect to the stabilizing assembly and the patient.

The stabilizing assembly is also capable of being released from, or releasing, the guide tube 20, 120, 222, 322 to allow the guide tube 20, 120, 222, 322 to be moved with respect to the stabilizing assembly. For example, when the guide tube 20, 120, 222, 322 is moved with respect to the stabilizing assembly, the distal end of the guide tube 20, 120, 222, 322 moves from one distal location towards a new distal location, which may be located with respect to a reference point (e.g., the location of the stabilizing assembly). Then, the stabilizing assembly may be re-secured to the guide tube 20, 120, 222, 322 (or vice versa). The stabilizing assembly may be configured-to be secured at varying positions along the guide tube 20, 120, 222, 322, and the positions may be predetermined, incremental, or freely selectable depending on the mechanism used to secure the stabilizing assembly to the guide tube 20, 120, 222, 322. As a result, the guide tube 20, 120, 222, 322 may be moved translationally with respect to the stabilizing assembly and the patient. Furthermore, the guide tube 20, 120, 222, 322 may include the retractor assembly 30, 40, 50, 60, which may move axially or radially with respect to the guide tube 20, 120, 222, 322.

The retractor assembly 30, 40, 50, 60 is configured to retract (or push against, apply a force to, move, etc.) a body organ, a wall of a body organ, a body lumen, or any other type of body tissue, e.g., through longitudinal displacement of the guide tube 20, 120, 222, 322, through longitudinal displacement-of the retractor assembly 30, 40, 50, 60, or-through radial expansion of the retractor assembly 30, 40, 50, 60 (e.g., inside a body lumen or body cavity). The retractor assembly 30, 40, 50, 60 may move with respect to a reference point, e.g., the location of the stabilizing assembly. The retractor assembly 30, 40, 50, 60 may be used to retract a body organ or other body tissue to release or deflate insufflation.

Other embodiments of the invention will be apparent to those skilled in the art from consideration of the specification and practice of the invention disclosed herein. It is intended that the specification and examples be considered as exemplary only, with a scope of the invention being indicated by the following claims.

## Claims

1. A medical device, comprising:
- an elongated member (26, 126, 226, 326); and
- a retractor assembly (30, 40, 50, 60) disposed on the elongated member near a distal end of the elongated member, the retractor assembly being configured to change from a collapsed configuration to an expanded configuration, the retractor assembly being configured to be movable within a body cavity when the retractor assembly is in an expanded configuration;
- a stabilizing assembly (70, 100, 110, 210, 310) configured to be releasably secured to the elongated member near a proximal end of the elongated member, the stabilizing assembly including a portion capable of being secured to a patient.

2. The medical device of claim 1, further including a linkage (36) extending through the elongated member (26, 126, 226, 326) from the distal end of the elongated member (26, 126, 226, 326) to a proximal end of the elongated member (26, 126, 226, 326); the retractor assembly (30, 40, 50, 60) being connected to the linkage (36) such that the linkage (36) moves the retractor assembly (30, 40, 50, 60) between the collapsed and expanded configurations.

3. The medical device of claim 1, wherein the retractor assembly (30, 40, 50, 60) includes at least one expandable member (32, 42, 52), the at least one expandable member (32, 42, 52) being changeable between an expanded configuration when the retractor assembly (30, 40, 50, 60) is in the expanded configuration and a collapsed configuration when the retractor assembly (30, 40, 50, 60) is in the collapsed configuration.

4. The medical device of claim 3, wherein the at least one expandable member (32, 42, 52) extends radially from the elongated member (26, 126, 226, 326) in the expanded configuration and lies substantially flat against the elongated member (26, 126, 226, 326) in the collapsed configuration.

5. The medical device of claim 3, wherein the at least one expandable member (32, 42, 52) includes a plurality of flanges (32) or loops (52).

6. The medical device of claim 3, wherein the at least one expandable member (32, 42, 52) includes an inflatable balloon (42), the balloon (42) being inflated when the retractor assembly (30, 40, 50, 60) is in the expanded configuration and deflated when the retractor assembly (30, 40, 50, 60) is in the collapsed configuration.

7. The medical device of claim 6, further including a linkage (36) extending longitudinally through the elongated member (26, 126, 226, 326) from the balloon to a proximal end of the elongated member (26, 126, 226, 326), the balloon (42) being connected to the linkage (36) such that a fluid may be transferred to and from the balloon (42) via the linkage (36).

8. The medical device of claim 1, wherein the portion of the stabilizing assembly (70, 100, 110, 210, 310) capable of being secured to the patient includes a mouthpiece (72, 172, 212, 312, 332) capable of being gripped by the patient.

9. The medical device of claim 1, wherein the stabilizing assembly (70, 100, 110, 210, 310) includes at least one of a portion (102) configured to form a friction fit with an outer surface of the elongated member (26, 126, 226, 326), a notch (92) for engaging with one of a plurality of rachet teeth (90) on the elongated member (26, 126, 226, 326), or two portions (112) connected by a hinge (114) that allows the portions to be in an open configuration and a closed configuration.

10. The medical device of claim 1, wherein the stabilizing assembly (70, 100, 110, 210, 310) further includes a lower portion (332) including a passageway (336) to allow air to pass therethrough.

11. The medical device of claim 1, wherein the elongated member (26, 126, 226, 326) is a flexible guide tube (20).

12. The medical device of claim 1, wherein the retractor assembly (30, 40, 50, 60) is configured to advance through an incision (10) in body tissue (8) when the retractor assembly (30, 40, 50, 60) is in the collapsed configuration.

13. The medical device of claim 12, wherein the retractor assembly (30, 40, 50, 60) is configured to retract the body tissue when the retractor assembly (30, 40, 50, 60) is in an expanded configuration.

## Patentansprüche

1. Medizinische Vorrichtung umfassend:
• ein langgestrecktes Element (26, 126, 226, 326); und
• eine Retraktoranordnung (30, 40, 50, 60), angeordnet auf dem langgestreckten Element nahe einem distalen Ende des langgestreckten Elementes, wobei die Retraktoranordnung konfiguriert ist, sich aus einer zusammen gefalteten Konfiguration zu einer expandierten Konfiguration zu verändern und die Retraktoranordnung konfiguriert ist, innerhalb eines Körperhohlraums bewegbar zu sein, wenn die Retraktoranordnung in einer expandierten Konfiguration ist;
• eine Stabilisierungsanordnung (70, 100, 110, 210, 310), die konfiguriert ist, auf lösbare Weise nahe einem proximalen Ende des langgestreckten Elementes befestigt zu werden und wobei die Stabilisierungsanordnung einen Abschnitt aufweist, der an einem Patienten befestigt werden kann.

2. Medizinische Vorrichtung gemäß Anspruch 1, ferner beinhaltend ein sich von dem distalen Ende des langgestreckten Elementes (26, 126, 226, 326) zu einem proximalen Ende des langgestreckten Elementes (26, 126, 226, 326) erstreckendes Verbindungselement (36); wobei die Retraktoranordnung (30, 40, 50, 60) derart mit dem Verbindungselement (36) verbunden ist, dass das Verbindungselement (36) die Retraktoranordnung (30, 40, 50, 60) zwischen der zusammen gefalteten und der expandierten Konfiguration bewegt.

3. Medizinische Vorrichtung gemäß Anspruch 1, wobei die Retraktoranordnung (30, 40, 50, 60) mindestens ein expandierbares Element (32, 42, 52) beinhaltet und das mindestens eine expandierbare Element (32, 42, 52) zwischen einer expandierten Konfiguration, wenn die Retraktoranordnung (30, 40, 50, 60) in der expandierten Konfiguration ist, und einer zusammen gefalteten Konfiguration, wenn die Retraktoranordnung (30, 40, 50, 60) in der zusammen gefalteten Konfiguration ist, veränderbar ist.

4. Medizinische Vorrichtung gemäß Anspruch 3, wobei das mindestens eine expandierbare Element (32, 42, 52) sich in der expandierten Konfiguration radial von dem langgestreckten Element (26, 126, 226, 326) erstreckt und in der zusammen gefalteten Konfiguration im wesentlichen flach an dem langgestreckten Element (26, 126, 226, 326) anliegt.

5. Medizinische Vorrichtung gemäß Anspruch 3, wobei das mindestens eine expandierbare Element (32, 42, 52) eine Vielzahl von Flanschen (32) oder Schlaufen (52) beinhaltet.

6. Medizinische Vorrichtung gemäß Anspruch 3, wobei das mindestens eine expandierbare Element (32, 42, 52) einen aufblasbaren Ballon (42) beinhaltet und der Ballon aufgeblasen ist, wenn die Retraktoranordnung (30, 40, 50, 60) in der expandierten Konfiguration ist, und entleert, wenn die Retraktoranordnung (30, 40, 50, 60) in der zusammen gefalteten Konfiguration ist.

7. Medizinische Vorrichtung gemäß Anspruch 6, ferner beinhaltend ein Verbindungselement (36), das sich in Längsrichtung von dem Ballon durch das langgestreckte Element (26, 126, 226, 326) hindurch zu einem proximalen Ende des langgestreckten Elementes (26, 126, 226, 326) erstreckt, wobei der Ballon (42) derart mit dem Verbindungselement (36) verbunden ist, dass über das Verbindungselement (36) ein Fluid zu und von dem Ballon (42) übertragen werden kann.

8. Medizinische Vorrichtung gemäß Anspruch 1, wobei der Abschnitt der Stabilisierungsanordnung (70, 100, 110, 210, 310), der an dem Patienten befestigt werden kann, ein Mundstück (72, 172, 212, 312, 332) beinhaltet, das von dem Patienten ergriffen werden kann.

9. Medizinische Vorrichtung gemäß Anspruch 1, wobei die Stabilisierungsanordnung (70, 100, 110, 210, 310) mindestens einen Abschnitt (102) beinhaltetet, der konfiguriert ist, einen Reibschluss mit einer äußeren Oberfläche des langgestreckten Elementes (26, 126, 226, 326) auszubilden, sowie eine Raste (92) zum Rasten mit einem einer Vielzahl von Klinkenzähnen (90) auf dem langgestreckten Element (26, 126, 226, 326) oder zwei Abschnitte (112), die mit einem Scharnier (114) verbunden sind, welches die Abschnitte in einer offenen Konfiguration und einer geschlossenen Konfiguration sein lässt.

10. Medizinische Vorrichtung gemäß Anspruch 1, wobei die Stabilisierungsanordnung (70, 100, 110, 210, 310) ferner einen unteren Abschnitt (332) beinhaltete, welcher einen Durchgang (336) enthält, der Luft durch ihn hindurch lässt.

11. Medizinische Vorrichtung gemäß Anspruch 1, wobei das langgestreckte Element (26, 126, 226, 326) ein flexibles Führungsrohr (20) ist.

12. Medizinische Vorrichtung gemäß Anspruch 1, wobei die Retraktoranordnung (30, 40, 50, 60) konfiguriert ist, sich durch einen Einschnitt (10) in Körpergewebe (8) vorwärts zu bewegen, wenn die Retraktoranordnung (30, 40, 50, 60) in der zusammen gefalteten Konfiguration ist.

13. Medizinische Vorrichtung gemäß Anspruch 12, wobei die Retraktoranordnung (30, 40, 50, 60) konfiguriert ist, das Körpergewebe zurück zu ziehen, wenn die Retraktoranordnung (30, 40, 50, 60) in einer expandierten Konfiguration ist.

## Revendications

1. Dispositif médical comprenant:
• un élément longitudinal (26, 126, 226, 326); et
• un ensemble rétracteur (30, 40, 50, 60), disposé sur l'élément longitudinal auprès d'une extrémité distale de l'élément longitudinal, l'ensemble rétracteur étant configuré à changer d'une configuration repliée à une configuration dilatée et l'ensemble rétracteur étant configuré à être mobile dans une cavité de corps quand l'ensemble rétracteur est dans une configuration dilatée;
• un ensemble stabilisateur (70, 100, 110, 210, 310), configuré à être fixé de façon amovible à l'élément longitudinal auprès d'une extrémité proximale de l'élément longitudinal, l'ensemble stabilisateur ayant une portion qui est capable à être fixée sur un patient.

2. Dispositif médical selon la revendication 1, comprenant en outre un élément de liaison (36) s'étendant de l'extrémité distale de l'élément longitudinal (26, 126, 226, 326) à une extrémité proximale de l'élément longitudinal (26, 126, 226, 326); l'ensemble rétracteur (30, 40, 50, 60) étant connecté à l'élément de liaison (36) de sorte que l'élément de liaison (36) change l'ensemble rétracteur (30, 40, 50, 60) entre la configuration repliée et la configuration dilatée.

3. Dispositif médical selon la revendication 1, dans lequel l'ensemble rétracteur (30, 40, 50, 60) inclut au moins un élément dilatable (32, 42, 52) et l'au moins un élément dilatable (32, 42, 52) est changeable entre une configuration dilatée quand l'ensemble rétracteur (30, 40, 50, 60) est dans la configuration dilatée, et une configuration repliée quand l'ensemble rétracteur (30, 40, 50, 60) est dans la configuration repliée.

4. Dispositif médical selon la revendication 3, dans lequel l'au moins un élément dilatable (32, 42, 52) s'étend de façon radiale à partir de l' élément longitudinal (26, 126, 226, 326) dans la configuration dilatée, et porte bien de manière essentiellement plaine contre l'élément longitudinal (26, 126, 226, 326) dans la configuration repliée.

5. Dispositif médical selon la revendication 3, dans lequel l'au moins un élément dilatable (32, 42, 52) inclut une pluralité de collerettes (32) ou de tirants (52).

6. Dispositif médical selon la revendication 3, dans lequel l'au moins un élément dilatable (32, 42, 52) inclut un ballonnet (42) gonflable et le ballonnet est gonflé quand l'ensemble rétracteur (30, 40, 50, 60) est dans la configuration dilatée, et dégonflé quand l'ensemble rétracteur (30, 40, 50, 60) est dans la configuration repliée.

7. Dispositif médical selon la revendication 6, incluant en outre un élément de liaison (36), qui s'étend longitudinalement à travers l'élément longitudinal (26, 126, 226, 326) à partir du ballonnet à une extrémité proximale de l'élément longitudinal (26, 126, 226, 326), le ballonnet (42) étant connecté à l'élément de liaison (36) de sorte qu'un fluide peut être transféré au et du ballonnet (42) via l'élément de liaison (36).

8. Dispositif médical selon la revendication 1, dans lequel la portion de l'ensemble stabilisateur (70, 100, 110, 210, 310) susceptible d'être fixée sur le patient inclut une embouchure (72, 172, 212, 312, 332) susceptible d'être saisie par le patient.

9. Dispositif médical selon la revendication 1, dans lequel l'ensemble stabilisateur (70, 100, 110, 210, 310) inclut au moins une portion (102) configurée à former un engagement par friction avec une surface extérieure d l'élément longitudinal (26, 126, 226, 326), une entaille (92) pour s'engager à un d'une pluralité de dents de blocage (90) sur l'élément longitudinal (26, 126, 226, 326), ou deux portions (112) connectées par une charnière (14) qui permet aux portions d'être dans une configuration ouverte et dans une configuration fermée.

10. Dispositif médical selon la revendication 1, dans lequel l'ensemble stabilisateur (70, 100, 110, 210, 310) inclut en outre une portion inférieure (332) qui inclut un chemin de passage (336) permettant à de l'air d'y passer.

11. Dispositif médical selon la revendication 1, dans lequel l'élément longitudinal (26, 126, 226, 326) est un tube de guidage flexible (20).

12. Dispositif médical selon la revendication 1, dans lequel l'ensemble rétracteur (30, 40, 50, 60) est configuré pour avancer à travers une incision (10) dans du tissu de corps (8) quand l'ensemble rétracteur (30, 40, 50, 60) est dans la configuration repliée.

13. Dispositif médical selon la revendication 12, dans lequel l'ensemble rétracteur (30, 40, 50, 60) est configuré de retirer le tissu de corps quand l'ensemble rétracteur (30, 40, 50, 60) est dans une configuration dilatée.
